# EUROPEAN PATENT SPECIFICATION

(11) **EP 2 484 612 B1**
(45) Date of publication and mention of the grant of the patent: **26.10.2016**
(21) Application number: 10820455.3
(22) Date of filing: 24.09.2010
(51) Int. Cl.: B65H 20/10, A61F 13/15, B65H 20/06

(54) **WEB CONVEYANCE DEVICE**
NETZFÖRDERVORRICHTUNG
DISPOSITIF DE CONVOYAGE DE NAPPE

(30) Priority: 30.09.2009 JP 2009229147
(43) Date of publication of application: 08.08.2012
(73) Proprietor: Unicharm Corporation, Ehime 799-0111 (JP)
(72) Inventor: YAMAMOTO, Hiroki, Kanonji-shi Kagawa 769-1602 (JP)
(74) Representative: Peter, Julian
(86) International application number: PCT/JP2010/066576
(87) International publication number: WO 2011/040338

(56) References cited:
- WO-A1-88/00922
- JP-A- 2001 019 070
- JP-A- 2007 117 646
- US-A1- 2006 289 692

## Description

### [technical Field]

The present invention relates to a web conveyor for conveying a web having a top surface on which workpieces are disposed at predetermined intervals in a machine direction along a flow direction of manufacturing steps for an absorbent article.

### [Background Art]

In the current field of manufacturing absorbent articles such as sanitary napkins and disposable diapers, techniques are desired for efficiently conveying soft absorbent articles without damage thereto. An apparatus for manufacturing absorbent articles desirably has a manufacturing line that is as linear as possible. In actuality however, an apparatus for manufacturing absorbent articles is constructed in a limited space, and so the conveying direction of the articles being conveyed (referred to hereinafter as "workpieces") is frequently changed. A web conveyor has been disclosed that conveys workpieces pressed against a pair of endless belts (see Patent Document 1).

However, the conventional web conveyor described above has the following problems. A belt that presses the workpieces from two surfaces has a non-continuous region In this region, the workpieces cannot be sufficiently pressed, and so the workpieces cannot be reliably conveyed.

For example, in a region where one of two belts pressing the surfaces of the workpieces is separated from a workpiece, this workpiece can stick to this separated belt and become wrapped around the belt. Furthermore, as the conveying speed is increased, the wind speed is also increased, so that there is a worry that the workpiece will rise up. These problems that can occur in the workpieces during the conveying lead to manufacturing defects and a drop in the manufacturing efficiency.

### [Prior Art Document]

### [patent Document]

Patent Document1: Patent No. 003647301

Further prior art defining in the technical field of the invention is disclosed in the patent application JP 2001 019070 A.

### [Summary of Invention]

An absorbent article manufacturing apparatus comprising a web conveyor of first aspect conveys a web in a machine direction along a flow direction of steps for manufacturing absorbent articles, the web having a top surface on which workpieces are arranged at predetermined intervals. The web conveyor includes a belt conveyor that includes a region for adsorbing a bottom surface of the web, and conveys the web in the machine direction; and a holding conveyor that holds at least top surfaces of the workpieces. The belt conveyor has a length in the machine direction greater than a length of the holding conveyor in the machine direction, and extends downstream from an end portion of the holding conveyor at least at a downstream side. The holding conveyor has a width arranged at least within a width of the workpieces. A boundary at which conveying conditions of the belt conveyor change exists between an upstream end portion and a downstream end portion of the holding conveyor. The web conveyor is arranged in the absorbent article manufacturing apparatus, which is formed by combining a plurality of units provided with mechanisms to perform at least a portion of the steps for manufacturing the absorbent articles, in a manner to straddle a boundary between the units. The boundary between the units is located between the upstream end portion and the downstream end portion of the holding conveyor, wherein the internal pressure of a downstream unit is greater than internal pressure of an upstream unit.

### [Brief Description of Drawings]

[Fig. 1]

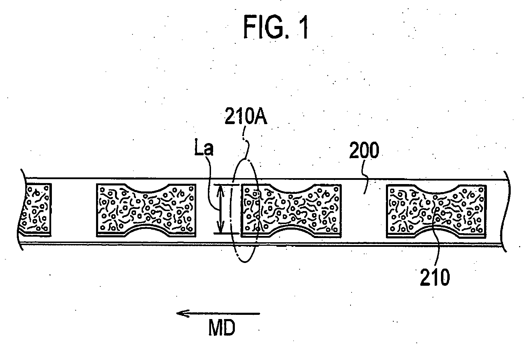

   Fig. 1 is a schematic view of an exemplary web conveyed by a web conveyor.
[Fig. 2]
   Fig. 2 is a perspective view of the web conveyor.
[Fig. 3] Fig. 3 (a) is a side view as seen from the direction of the arrow A shown in Fig. 2.
Fig. 3 (b) is a top view as seen from the direction of the arrow B shown in Fig. 2.
[Fig. 4]

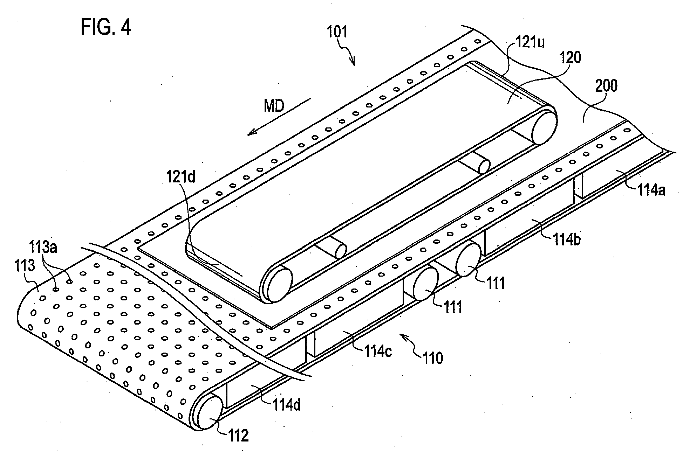

   Fig. 4 is a perspective view of a web conveyor.
[Fig. 5]

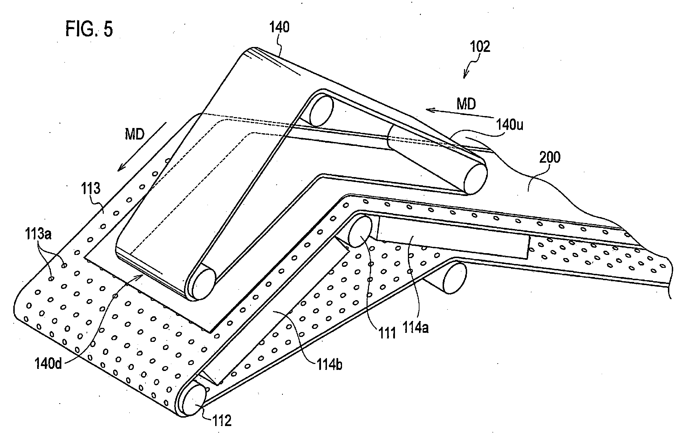

   Fig. 5 is a perspective view of a web conveyor according to a modification.
[Fig. 6]

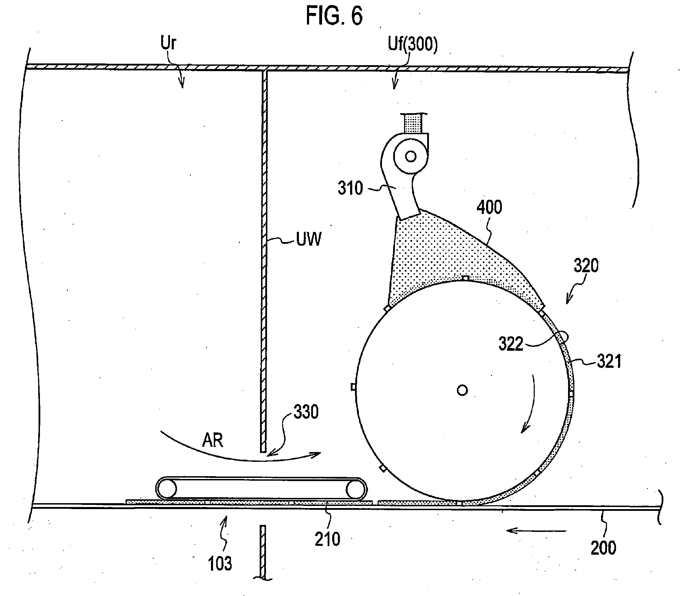

   Fig. 6 is a view for illustrating a desirable location for arranging the web conveyor within a piece of equipment according to the invention.
[Fig. 7]

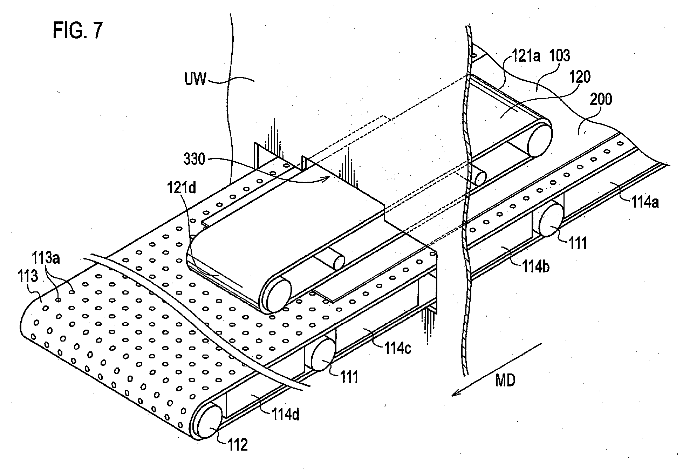

   Fig. 7 is a perspective view of a web conveyor according to the invention.

### [Description of Embodiments]

An embodiment of a web conveyer according to the present invention will be described with reference to the drawings 6 to 7. Note that, in the following description of the drawings, the same or similar numerals denote the same or similar portions. In addition, it should be noted that the drawings are schematic and ratios of dimensions and the like are different from actual ones. Therefore, specific dimensions and the like should be determined in consideration of the following description. Moreover, as a matter of course, the drawings also include portions having different dimensional relationships and ratios from each other.

Fig. 1 is a schematic view of an exemplary web 200 conveyed by a web conveyor. The web 200 is a continuous body that makes up an absorbent article or the like.

The web 200 is a product such as an absorbent article that is in the process of being manufactures, and is conveyed in a machine direction MD as it progresses through the manufacturing steps. Workpieces 210 are arranged on the top surface of the web 200 at predetermined intervals. Region 210A represents the portion of a workpiece 210 that leads in the machine direction MD. The length in the width direction of the region 210A is represented as La. In a case where the product is a diaper, for example, the web 200 is a top sheet arranged on the skin contact surface side or a back sheet arranged on the clothing contact surface side, and the workpieces are absorbers. The web 200 is conveyed as it progresses through the manufacturing steps in the machine direction MD by a web conveyor 100 described below.

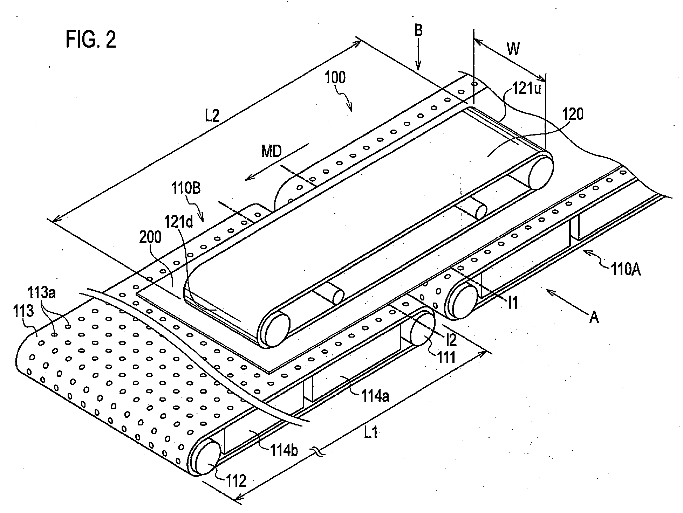

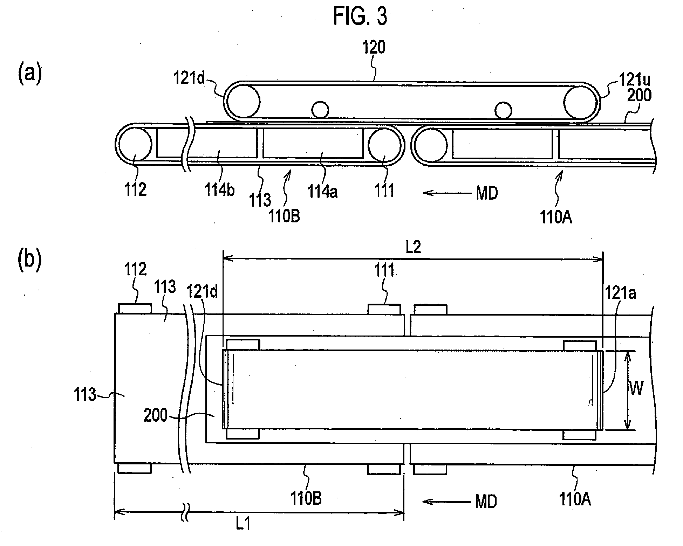

Fig. 2 is a perspective view illustrating the web conveyor 100. Fig. 3 (a) is a side view as seen from the direction of the arrow A shown in Fig. 2. Fig. 3 (b) is a top view as seen from the direction of the arrow B shown in Fig. 2.

The web conveyor 100 includes belt conveyors 110A and 110B that convey the web 200 in the machine direction MD, and a holding conveyor 120 that holds the top surface of the web 200. The holding conveyor 120 presses the top surface of the web 200 against the conveyor belts 110A and 110B.

The length L1 of the belt conveyors 110A, 110B, in the machine direction MD is greater than the length L2 of the holding conveyor 120 in the machine direction MD. The length L2 of the holding conveyor 120 is greater than the interval between workpieces 210, i.e. the work pitch. The belt conveyor 110B includes a roller 111 disposed upstream in the machine direction MD, a roller 112 disposed downstream, and an endless belt that is wrapped around the rollers 111 and 112. The endless belt 113 has a plurality of through-holes 113a formed therein.

An imaginary line 11 that is orthogonal to the machine direction MD and parallel to the conveying surface in the belt conveyor 110A is parallel to an imaginary line 12 of the belt conveyor 110B that is oriented in the same manner. In other words, the conveying surfaces of the belt conveyors 110A, 110B, adjacently disposed are flat, without bending in the machine direction MD.

The belt conveyor 110B includes a plurality of suction boxes 114a, 114b, that suck external air through the through-holes 113a. As a result, the belt conveyors 110A, 110B, can convey the web 200 while adsorbing the bottom surface thereof The belt conveyor 110A has the same configuration as the belt conveyor 110B.

The holding conveyor 120 includes an end portion 121u that is upstream in the machine direction MD and an end portion 121d that is downstream. The width w of the holding conveyor 120 is less than at least the width La (see Fig. 1) of the workpieces 210, and can be any length that can at least be arranged within the width La of the workpieces 210. The belt conveyor 110B extends father downstream than the end portion 121d of the holding conveyor 120 at least at the downstream side in the machine direction MD.

As shown in Figs. 2 and 3, in the web conveyor 100, there is a boundary between the upstream end portion 121u and the downstream end portion 121d of the holding conveyor 120, the boundary at which the conveying condition of the web 200 changes. Specifically, the boundary between the belt conveyor 110A and the belt conveyor 110B is located between the downstream end portion 121d and the upstream end portion 121u of the holding conveyor 120. In other words, the holding conveyor 120 that presses on the top surface of the web 200 is disposed at the boundary between the belt conveyor 110A and the belt conveyor 110B.

By providing the holding conveyor 120 at the boundary between the belt conveyor 110A and the belt conveyor 110B in the web conveyor 100, the top surfaces of the web 200 and the workpieces 210 are pressed, and so the workpieces 210 being conveyed are prevented from rising up from the web 200.

With this web conveyor 100, the the leading end of the web 200 is prevented from sticking and wrapping around the belt conveyor 110A at the boundary between the belt conveyor 110A and the belt conveyor 110B, which are the two belts pressing the top surfaces of the workpieces 210. Thus, the web 200 and the workpieces 210 can be reliably passed between the conveyors.

Furthermore, with the web conveyor 100, even when the wind pressure on a workpiece 210 increases due to an increase in the conveying speed, rising of the workpiece 210 from the web 200 or floating of the web 200 from the conveying surface can be prevented at locations where the web 200 and the workpieces 210 cannot be adsorbed to the conveying surface, such as the boundary between the belt conveyor 110A and the belt conveyor 110B. Furthermore, the web conveyor 100 can prevent problems with the workpieces during the conveying to enable reliable conveying, thereby preventing manufacturing defects and a drop in manufacturing efficiency.

In Figs. 1 to 3, although not shown, the holding conveyor 120 may be provided with an endless belt having through-holes and a suction box. The suction box is arranged between the end portion 121u and the end portion 121d. As a result, the holding conveyor 120 can convey the web 200 while adsorbing the top surface thereof. In this case, the space between the belt conveyor 110A and the belt conveyor 110B is longer than the length of the workpieces 210 in the machine direction MD. Sagging of the web 200 due to the weight of the workpieces 210 can be prevented even in a case where the space between the belt conveyor 110A and the belt conveyor 110B is longer than the length of the workpieces 210 in the machine direction MD. Thus, the web 200 and the workpieces 210 can be reliably conveyed. The holding conveyor 120 provided with the suction mechanism is particularly useful to convey the workpieces 210 only.

The following describes a web conveyor 101. Fig. 4 is a perspective view of the web conveyor 101.

The web conveyor 101 has boundaries between suction boxes 114a, 114b, 114c, 114d between the upstream end portion 121u and the downstream end portion 121d of the holding conveyor 120. Specifically, the holding conveyor 120 is arranged to press on the top surface of the web 200 at a boundary between the suction boxes 114b, 114c, of the belt conveyor 110.

Since a common suction box has a suitable size for achieving suction to reliably suck the web 200, a plurality of suction boxes are required to exert suction over the entire region of the belt conveyor 110. Therefore, there is a case where the conveying surface of the belt conveyor 110 includes locations which cannot adsorb the web 200 and the workpieces 210.

With the web conveyor 101 shown in Fig. 4, on the other hand, the web 200 and the workpieces 210 are pressed on their top surfaces by arranging the holding conveyor 120 over the boundary between the suction boxes 114b, 114c, of the belt conveyor 100. Therefore, rising of the workpieces 210 from the web 200 or floating of the web 200 from the conveying surface can be prevented at the locations in which the web 200 and the workpieces 210 cannot be adsorbed to the conveying surface.

The following describes a web conveyor 102. Fig. 5 is a perspective view of the web conveyor 102. In the web conveyor 102 shown in Fig. 6, the conveying surface is curved at the boundary between the suction boxes so as to cause the top surface of the web 200 to protrude. The curved portion is at the boundary between suction boxes. In the web conveyor 102, a holding conveyor 140 is arranged at the boundary between the suction boxes 114a, 114b, of the belt conveyor 110. The boundary between the suction boxes is located between an upstream end portion 140u and a downstream end portion 140d of the holding conveyor 140.

At locations where the conveying surface is curved to cause the top surface of the web 200 to protrude, such as corner portions of the conveying path where suction boxes cannot be disposed, particularly at locations where there is a change between an ascending surface and a descending surface, the workpieces 210 are more easily affected by wind resistance, causing the leading ends of the workpieces 210 to float up.

To prevent this floating, in the web conveyor 102 shown in Fig. 5, the holding conveyor 140 is provided at the location of the boundary between the suction boxes 114a, 114b, in which the conveying surface is curved to cause the top surface of the web 200 to protrude. Accordingly, rising, floating, or the like of the web 200 and the workpieces 210 can be prevented, thereby ensuring reliable conveying of the web 200 and the workpieces 210.

### (Embodiment according to the invention)

The following describes a web conveyor 103 according to the present invention. Fig. 6 is a view for illustrating a desirable location for arranging the web conveyor 103 within a piece of equipment.

The apparatus (equipment) for manufacturing absorbent articles is divided into units that represent a portion of a manufacturing step or a plurality of steps. The web is conveyed between units where predetermined manufacturing steps are performed, in order to produce a final product.

Fig. 6 shows a portion of equipment for manufacturing absorbent articles. Fig. 6 shows a unit Uf and a unit Ur. The unit Uf and the unit Ur are connected. Specifically, the unit Uf is an absorber forming unit 300 that forms an absorber.

The absorber forming unit 300 includes a discharger 310 that discharges a ground pulp 400 and a drum 320 that has a suction mechanism for sucking up the discharged pulp 400. A mold 321 is formed in the drum 320, and mesh or the like is provided on a bottom 322 of the mold 321 in order to adsorb the pulp 400. The absorbers that are layered within the mold 321 by the drum 320 are arranged on the web 200 at predetermined intervals.

In the Uf unit (absorber forming unit 300) shown in Fig. 6, noise is generated by the suction, and so a wall Uw, which may be soundproof, is formed. The wall Uw has an opening 330 through which the conveying path of the web 200 passes.

Since the unit Uf is provided with the drum 320 having the suction mechanism, the internal pressure of the unit Uf is lower than that of the unit Ur. Therefore, airflow AR occurs through the opening 330 from the unit Ur toward the unit Uf. Accordingly, the wind pressure on the web 200 and the workpieces 210, which are being conveyed from the unit Uf to the unit Ur, is greater than in a normal conveying path. Thus, it is assumed that the web 200 and the workpieces 210 are more likely to rise up.

On the other hand, in the present embodiment, as shown in Fig. 6, the web conveyor 103 is disposed at the opening 330. Fig. 7 is a perspective view of the web conveyor 103.

As shown in Fig. 7, in the web conveyor 103, the holding conveyor 120 is disposed at the boundary (opening 330) between the unit Uf having a low internal pressure and the unit Ur, whose internal pressure is higher than that of the unit Uf. Accordingly, rising, floating, or the like of the web 200 and the workpieces 210 can be prevented at the opening 330 in which the wind pressure is high, and so the web 200 and the workpieces 210 can be reliably conveyed.

### (Another Embodiment)

As described above, the details of the embodiment of the present invention has been exemplarily disclosed. However, it should not be understood that the description and drawings which constitute part of this disclose limit the present invention.
Based on this disclosure, various alternative embodiments, examples, and operation techniques are apparent to those skilled in the art, as defined within the appended claims.

In the above disclosure, the holding conveyor 120 presses the top surfaces of the web 200 and the workpieces 210 toward the belt conveyors 110A, 110B.
However, the holding conveyor 120 may have any configuration that enables reliable conveying of the web 200 and the workpieces 210. For example, the belt conveyor can have a suction mechanism that can convey the web 200 and the workpieces 210 while adsorbing them.

In the above disclosure, the suction boxes were used as an example of a mechanism for sucking the bottom surface of the web 200. However, such a mechanism is not limited to the suction boxes and any mechanism capable of adsorbing the web 200 can be used.

### [Industrial Applicability]

According to the present invention, provided is a web conveyor that can prevent ripping of workpieces and skew of workpieces relative to the web while conveying the workpieces in a machine direction along a flow direction of manufacturing steps for an absorbent article, and can thereby reliably convey the workpieces.

## Claims

1. Absorbent article manufacturing apparatus comprising: a web conveyor (100) for conveying a web (200) in a machine direction (MD) along a flow direction of steps for manufacturing absorbent articles, the web (200) having a top surface on which workpieces (210) are arranged at predetermined intervals, the web conveyor (100) comprising:
a belt conveyor (110) that includes a region for adsorbing a bottom surface of the web, and conveys the web in the machine direction (MD); and
a holding conveyor (120) that holds at least top surfaces of the workpieces (210), wherein:
the belt conveyor (110) has a length in the machine direction (MD) greater than a length of the holding conveyor (120) in the machine direction (MD), and extends downstream from an end portion of the holding conveyor (120) at least at a downstream side;
the holding conveyor (120) has a width arranged at least within a width of the workpieces (210); and
a boundary at which conveying conditions of the belt conveyor (110) change exists between an upstream end portion and a downstream end portion of the holding conveyor (120)
the web conveyor (103) is arranged in the absorbent article manufacturing apparatus, which is formed by combining a plurality of units (300, Uf, UR) provided with mechanisms to perform at least a portion of the steps for manufacturing the absorbent articles, in a manner to straddle a boundary between the units;
the boundary between the units is located between the upstream end portion and the downstream end portion of the holding conveyor (120);
**characterized by**
internal pressure of a downstream unit (UR) is greater than internal pressure of an upstream unit (Uf).

2. Absorbent article manufacturing apparatus according to Claim 1, wherein:
a wall (Uw) is disposed between the upstream unit and the downstream unit;
the wall (Uw) is provide with an opening for passing from the unit to the unit, the web (200) having the top surface on which the workpieces (200) are arranged at predetermined intervals; and
the belt conveyor (110) and the holding conveyor (120) pass through the opening.

3. Absorbent article manufacturing apparatus according to Claim 1 or Claim 2, wherein:
the workpieces (200) are absorbers for making up the absorbent articles; and
the upstream unit includes an absorber manufacturing apparatus for manufacturing the absorbers.

## Patentansprüche

1. Vorrichtung zur Herstellung von saugfähigen Artikeln umfassend: Einen Netzförderer (100) zum fördern eines Netzes (200) in eine Maschinenrichtung (MD) entlang einer Flussrichtung von Schritten zur Herstellung saugfähiger Artikel, wobei das Netz (200) eine obere Oberfläche, auf welcher Werkstücke (210) in vorbestimmten Intervallen angeordnet sind, aufweist und wobei der Netzförderer (100) umfasst:
einen Gurtförderer (110) mit einem Bereich zur Adsorption einer unteren Oberfläche des Netzes, der das Netz in die Maschinenrichtung (MD) fördert,
einen Aufnahmeförderer (120) der zumindest obere Oberflächen der Werkstücke (210) aufnimmt, wobei:
der Gurtförderer (110) eine Länge in die Maschinenrichtung (MD) größer als eine Länge des Aufnahmeförderers (120) in die Maschinenrichtung (MD) aufweist, und sich stromabwärts von einem Endabschnitt des Aufnahmeförderers (120), auf zumindest einer stromabwärtsliegenden Seite, erstreckt;
der Aufnahmeförderer (120) eine Breite aufweist, die zumindest innerhalb einer Breite der Werkstücke (210) angeordnet ist; und
eine Grenze, bei der sich die Förderbedingungen des Gurtförderers (110) ändern, zwischen einem stromaufwärtsliegenden Endabschnitt und einem stromabwärtsliegenden Endabschnitt des Aufnahmeförderers (120) der Netzförderer (103) in einer Weise in der saugfähiger-Artikel-Herstellungsvorrichtung angeordnet ist, welche durch kombinieren einer Vielzahlt von Einheiten (300, Uf, UR), welche mit Mechanismen zur Durchführung von zumindest einem Teil der Schritte zur Herstellung des saugfähigen Artikels versehen sind, gebildet wird, dass eine Grenze zwischen den Einheiten überbrückt wird;
die Grenze zwischen den Einheiten liegt zwischen den stromaufwärtsliegenden Endabschnitten und den stromabwärtsliegenden Endabschnitten des Aufnahmeförderers (120);
**dadurch gekennzeichnet, dass**
der innere Druck einer stromabwärtsliegenden Einheit (UR) größer ist als der innere Druck einer stromaufwärtsliegenden Einheit (Uf).

2. Vorrichtung zur Herstellung von saugfähigen Artikeln gemäß Anspruch 1, wobei:
eine Mauer (Uw) zwischen der stromaufwärtsliegenden Einheit und der stromabwärtsliegenden Einheit angeordnet ist;
die Mauer (Uw) eine Öffnung, zum passieren von der Einheit zu der Einheit, aufweist, das Netz (200) die obere Oberfläche aufweist, auf der die Werkstücke (200) in vorbestimmten Intervallen angeordnet sind; und
der Gürtelförderer (110) und der Aufnahmeförderer (120) die Öffnung durchlaufen.

3. Vorrichtung zur Herstellung von saugfähigen Artikeln gemäß Anspruch 1 oder Anspruch 2, wobei:
die Werkstücke (200) Absorber für die Herstellung der saugfähigen Artikel sind; und
die stromaufwärtsliegende Einheit eine Absorberherstellungsvorrichtung zur Herstellung der Absorber umfasst.

## Revendications

1. Appareil de fabrication d'articles absorbants comprenant : un convoyeur de nappe (100) pour convoyer une nappe (200) dans une direction de la machine (MD) le long d'une direction de flux d'étapes de fabrication d'articles absorbants, la nappe (200) ayant une surface supérieure sur laquelle des pièces à usiner (210) sont placées à des intervalles prédéterminés, le convoyeur de nappe (100) comprenant :
un convoyeur à courroie (110) qui comporte une région pour retenir par adsorption une surface inférieure de la nappe, et convoie la nappe dans la direction de la machine (MD) ; et
un convoyeur de maintien (120) qui maintient au moins des surfaces supérieures des pièces à usiner (210), dans lequel :
le convoyeur à courroie (110) a une longueur dans la direction de la machine (MD) plus grande qu'une longueur du convoyeur de maintien (120) dans la direction de la machine (MD), et s'étend vers l'aval depuis une partie d'extrémité du convoyeur de maintien (120) au moins sur un côté aval ; le convoyeur de maintien (120) a une largeur agencée au moins en-deçà d'une largeur des pièces à usiner (210) ; et
une frontière, à laquelle des conditions de convoyage du convoyeur à courroie (110) changent, existe entre une partie d'extrémité amont et une partie d'extrémité aval du convoyeur de maintien (120)
le convoyeur de nappe (103) est agencé dans l'appareil de fabrication d'articles absorbants, qui est formé en combinant une pluralité d'unités (300, Uf, UR) équipées de mécanismes pour effectuer au moins une partie des étapes pour fabriquer les articles absorbants, d'une manière à chevaucher une frontière entre les unités ; la frontière entre les unités est agencée entre la partie d'extrémité amont et la partie d'extrémité aval du convoyeur de maintien (120);
**caractérisé en ce que**
une pression interne de l'unité aval (UR) est plus grande qu'une pression interne d'une unité amont (Uf).

2. Appareil de fabrication d'articles absorbant selon la revendication 1, dans lequel :
une paroi (Uw) est disposée entre l'unité amont et l'unité aval ;
la paroi (Uw) est équipée d'une ouverture pour passer de l'unité à l'unité, la nappe (200) ayant la surface supérieure sur laquelle les pièces à usiner (200) sont agencées à des intervalles prédéterminés ; et le convoyeur à courroie (110) et le convoyeur de maintien (120) passent à travers l'ouverture.

3. Appareil de fabrication d'articles absorbants selon la revendication 1 ou la revendication 2, dans lequel :
les pièces à usiner (200) sont des absorbeurs pour composer les articles absorbants ; et
l'unité amont comporte un appareil de fabrication d'absorbeurs pour la fabrication des absorbeurs.
